(19) Europäisches Patentamt
European Patent Office
Office européen des brevets

(11) Veröffentlichungsnummer: **0 214 550 B1**

(12) # EUROPÄISCHE PATENTSCHRIFT

(45) Veröffentlichungstag der Patentschrift: **19.09.90**

(51) Int. Cl.⁵: **C07K 5/06**, C07K 1/00, C12P 21/02

(21) Anmeldenummer: **86111843.8**

(22) Anmeldetag: **27.08.86**

(54) **Verfahren zur Herstellung von Aspartam und Mittel zu seiner Durchführung.**

(30) Priorität: **09.09.85 DE 3532027**

(43) Veröffentlichungstag der Anmeldung: **18.03.87 Patentblatt 87/12**

(45) Bekanntmachung des Hinweises auf die Patenterteilung: **19.09.90 Patentblatt 90/38**

(84) Benannte Vertragsstaaten: **BE CH DE FR GB IT LI NL**

(56) Entgegenhaltungen:
EP-A- 0 035 047
EP-A- 0 075 160
EP-A- 0 099 585
EP-A- 0 102 483
EP-A- 0 143 881
EP-A- 0 149 594
EP-A- 0 169 937
EP-A- 0 170 148
DE-A- 2 801 238
GB-A- 1 339 101
GB-A- 2 160 870
JP-A- 5 484 522
US-A- 3 941 831

TETRAHEDRON LETTERS, Nr. 28, 1979, Seiten 2611-2612, Pergamon Press Ltd, GB; Y. ISOWA et al.: "The thermolysin-catalyzed condensation reaction of N-substituted aspartic and glutamic acids with phenylalanine alkyl esters"
HOUBEN-WEYL: "Methoden der organischen Chemie", Band E5, Carbonsäuren und Carbonsäure-Derivate, 1985, Seiten 702-705, Georg Thieme Verlag, Stuttgart, DE; H. PIELARTZIK et al.: "Aus Carbonsäure-estern"

(73) Patentinhaber: **HOECHST AKTIENGESELLSCHAFT, Postfach 80 03 20, D-6230 Frankfurt am Main 80(DE)**

(72) Erfinder: **Keller, Reinhold, Dr., Wiesenweg 5, D-6232 Bad Soden am Taunus(DE)**
Erfinder: **Schlingmann, Merten, Dr., Schneidhainer Strasse 32a, D-6240 Königstein/Taunus(DE)**
Erfinder: **Platen, Martin, Dr., Krautgartenweg 6, D-6000 Frankfurt am Main 50(DE)**

(56) Entgegenhaltungen: (Fortsetzung)

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

In der deutschen Offenlegungsschrift 2 801 238 (US 4 165 311; 4 256 836; 4 436 925) wird ein Verfahren zur Herstellung von Additionsverbindungen beschrieben, die aus einem N-geschützten Monoaminocarbonsäureester einerseits und einem Dipeptid andererseits bestehen, wobei das Dipeptid wiederum aus diesem Monoaminocarbonsäureester und einer Monoaminodicarbonsäure in einer enzymatischen Veresterungsreaktion hergestellt wurde. Die Additionsverbindung ist im Reaktionsmedium unlöslich und wird abgetrennt. Das eigentliche Ziel dieser Umesterung ist jedoch nicht die Herstellung der Additionsverbindung, sondern das ungeschützte Dipeptid selbst. Es sind in der Literatur, unter anderem auch in der obengenannten Deutschen Offenlegungsschrift, mehrere Methoden beschrieben, die die Gewinnung des Dipeptids durch Zersetzung der Additionsverbindung zum Gegenstand haben.

Das Verfahren findet seine praktische Anwendung z.B. in der Herstellung des Süßstoffs Aspartam, ein Dipeptidester, der aus Asparaginsäure und D- und/oder L-Phenylalaninmethylester als Ausgangsprodukten hergestellt wird. Die Asparaginsäure wird enzymatisch mit dem L-Phenylalaninmethylester verknüpft, während sich überschüssiger D-Phenylalaninmethylester an das Dipeptid zur Adduktbildung anlagert. In einem weiteren Reaktionsschritt wird der Aspartylphenylalaninmethylester aus dem Addukt freigesetzt.

In Houben-Weyl (1985, E5, 702–705) werden eine Reihe allgemeiner Umesterungsreaktionen beschrieben. Unter den dort genannten Bedingungen bleiben die chiralen Zentren erhalten. Dies trifft jedoch nicht auf Umesterungen von Phenylalkylestern zu. Bei den Phenylalkylestern tritt in der Regel eine Racemisierung der zwei chiralen Kohlenstoffatome auf (US 3 941 831; J OS 54-84 522; Houben-Weyl, Synthese von Peptiden I, 1974, 34–37).

Es hat sich jedoch überraschend gezeigt, daß die enzymatische Verknüpfung mit höheren Alkylestern als den Methyl- oder Ethylestern des D-,L-Phenylalanins, wesentliche Vorteile in der weiteren Aufarbeitung bewirkt.

Es wurde außerdem gefunden, daß die entsprechenden Dipeptidalkylester der höheren Alkohole durch eine schnelle, schonende Umesterung in wasserfreiem methanolischen Medium in Gegenwart basischer Katalysatoren, wie z.B. Alkanolate oder Anionenaustauscher, quantitativ in die gewünschte Methylesterverbindung überführt werden können, ohne daß unter diesen Bedingungen eine Racemisierung an den beiden chiralen Kohlenstoffatomen erfolgt. Dies war besonders überraschend, da in der veröffentlichten japanischen Patentanmeldung 79 84522 unter den gleichen Bedingungen die Racemisierung von optisch aktiven α-Aminosäurealkylestern beschrieben wird.

Die Erfindung betrifft somit:

1. Ein Verfahren zur Herstellung der Verbindung der allgemeinen Formel III,

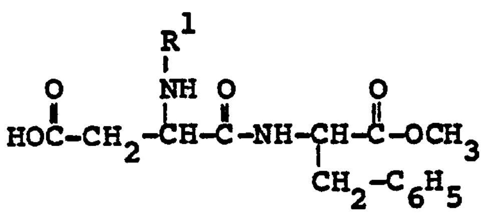

III

in der $R^1$ eine Aminoschutzgruppe ist, das dadurch gekennzeichnet ist, daß die Verbindung der allgemeinen Formel II,

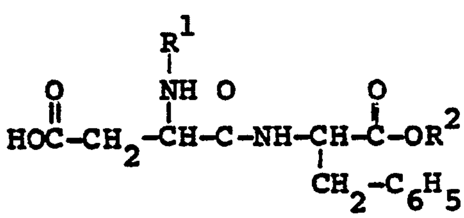

II

in der $R^1$ eine Aminoschutzgruppe und $R^2$ eine Alkylgruppe mit 3-7 Kohlenstoffatomen bedeuten, in wasserfreiem Methanol umgeestert wird.

2. Die Verbindung der allgemeinen Formel II,

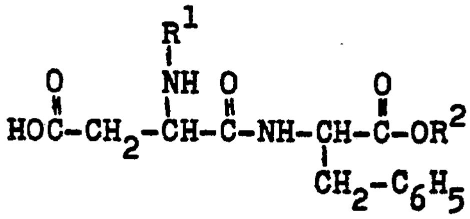

II

in der $R^1$ eine Aminoschutzgruppe und $R^2$ eine Alkylgruppe mit 3-7 Kohlenstoffatomen bedeuten.

3. Die Additionsverbindung der allgemeinen Formel I,

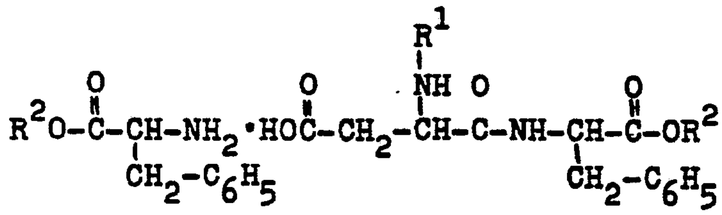

I

in der $R^1$ eine Aminoschutzgruppe und $R^2$ eine Alkylgruppe mit 3-7 Kohlenstoffatomen bedeuten.

4. Ein Verfahren zur Herstellung der Additionsverbindung der allgemeinen Formel I, in der $R^1$ eine Aminoschutzgruppe und $R^2$ eine Alkylgruppe sind, durch Verknüpfung einer N-geschützten Asparaginsäure mit einem D-,L-Phenylalaninalkylester oder L-Phenylalaninalkylester in Gegenwart einer Protease, in einem pH-Bereich, in dem die Protease enzymatisch aktiv ist, das dadurch gekennzeichnet ist, daß man einen D-,L-Phenylalaninalkylester oder L-Phenylalaninalkylester verwendet, dessen Alkylgruppe 3-7 Kohlenstoffatome hat.

5. Ein Verfahren zur Herstellung der Verbindung der allgemeinen Formel II, in der $R^1$ eine Aminoschutzgruppe und $R^2$ eine Alkylgruppe sind, durch Zersetzung der Additionsverbindung der allgemeinen Formel I, in der $R^1$ und $R^2$ die obengenannte Bedeutung haben, in wäßrig-saurer Lösung das dadurch gekennzeichnet ist, daß man eine Additionsverbindung der allgemeinen Formel I einsetzt, in der $R^1$ die oben genannte Bedeutung hat und $R^2$ eine Alkylgruppe mit 3-7 Kohlenstoffatomen bedeutet.

In den allgemeinen Formeln I, II und III ist $R^1$ eine Aminoschutzgruppe, wobei eine Schutzgruppe vom Urethantyp bevorzugt ist, insbesondere eine Benzyloxycarbonylgruppe oder eine tertiär-Butyloxycarbonylgruppe. In den Formeln I und II ist $R^2$ eine Alkylgruppe mit 3-7 Kohlenstoffatomen, mit n-Propyl, iso-Propyl, n-Butyl und n-Pentyl als besonders bevorzugten Gruppen.

Die Herstellung der Additionsverbindung der allgemeinen Formel I und die Freisetzung der Verbindung der allgemeinen Formel II aus dieser Additionsverbindung erfolgt analog der Deutschen Offenlegungsschrift 2 801 238. Man setzt die N-geschützte L-Asparaginsäure mit einem D-,L-Phenylalaninalkylester oder L-Phenylalaninalkylester, dessen Alkylgruppe 3-7 Kohlenstoffatome hat, in wäßrigem Medium mit Hilfe einer Protease zu der Verbindung der allgemeinen Formel II um, die sich unter den Reaktionsbedingungen mit überschüssigem Phenylalaninalkylester zu der Additionsverbindung der allgemeinen Formel I verknüpft, die dann ausfällt und abgetrennt werden kann. Als Protease wird bevorzugt ungereinigtes Thermolysin eingesetzt. Die Reaktion verläuft vorteilhaft in wäßriger Lösung bei einem pH-Wert von 5,0 - 6,0, wobei die N-geschützte Asparaginsäure zusammen mit dem Enzym vorgelegt wird, und der D-,L-Phenylalaninalkylester in Form einer konzentrierten wäßrigen Lösung im Verlauf der Reaktion zudosiert wird. Ebenfalls von Vorteil ist das Zudosieren einer hochkonzentrierten wäßrigen Lösung von N-geschützter Asparaginsäure und D-,L-Phenylalaninalkylester in eine wäßrige Vorlage von Enzym.

Eine weitere Möglichkeit zur Herstellung einer Additionsverbindung wird in der Patentanmeldung P 3 517 361.0 vorgeschlagen. Die Verbindung der Formel II reagiert mit einem im Reaktionsgemisch vorhandenen Amin zum Addukt.

Die Verbindung der allgemeinen Formel II wird durch Zugabe einer starken Säure, bevorzugt Salzsäure, in wäßrigem Medium aus der erfindungsgemäßen Additionsverbindung freigesetzt. Der dabei ebenfalls freigesetzte Phenylalaninalkylester kann aus alkalisch-wäßriger Lösung mit einem organischen Lösungsmittel, bevorzugt Diethylether, extrahiert werden und anschließend durch Einleiten gasförmiger Salzsäure als Hydrochlorid ausgefällt werden.

Die Verwendung der ($C_3$-$C_7$)-Alkylesterverbindungen hat folgende Vorteile im Vergleich zu den niedrigeren Homologen:

Der ($C_3$-$C_7$)-D-,L-Phenylalaninalkylester bzw. die entsprechende L-Verbindung zeigt eine größere Stabilität gegenüber chemischer Hydrolyse. Die entsprechende Additionsverbindung ist leichter aus dem Reaktionsgemisch abzutrennen. Nach Aufspaltung des Addukts sind die ($C_3$-$C_7$)-D-,L-Phenylala-

ninalkylester bzw. die entsprechende L-Verbindung einfacher und in quantitativer Ausbeute wieder zurückzugewinnen.

Am Ende der beschriebenen Reaktionsfolge muß die Verbindung der allgemeinen Formel II zu dem entsprechenden Methylester umgeestert werden, um das Aspartam zu erhalten.

Zur Umesterung wird der Dipeptidester in wasserfreiem Methanol, unter Zugabe eines Alkalimetallalkoholats als Katalysator, gelöst. Wenn die Reaktion in Gegenwart von Natrium- oder Kaliummethanolat durchgeführt wird, verläuft die Umesterung besonders vorteilhaft. Der Katalysator wird in 1- bis 5facher molarer Menge, bezogen auf den Dipeptidester, eingesetzt. Bevorzugt ist jedoch die 1,1- bis 2fache molare Menge. Die Reaktion läuft bei einer Temperatur von -20 °C bis 50 °C ab, vorzugsweise bei 0 °C bis 40 °C, insbesondere bei 18 °C bis 25 °C. Die Reaktionsdauer beträgt unter Anwendung des besonders bevorzugten Temperaturbereichs nur 10 bis 15 Minuten. Die Durchführung der Reaktion bei einer mäßigen Temperatur, wie z.B. Raumtemperatur, bietet nicht nur den Vorteil, daß Energie gespart wird, sondern auch daß Nebenreaktionen unterdrückt werden. Falls der Dipeptidmethylester isoliert werden soll, kann er nach dem Einengen der Reaktionslösung in eine Säure eingetragen werden und anschließend aus einem geeigneten Lösungsmittel auskristallisiert werden.

Die folgenden Beispiele geben eine weitere detaillierte Beschreibung der Erfindung. Die Prozentangaben beziehen sich auf das Gewicht.

Beispiel 1

Herstellung von N-geschützem L-Aspartylphenylalaninpropylester-Addukt

53,0 g (0,2 Mol) L-N-Benzyloxycarbonylasparaginsäure und 1 g Calciumacetat werden in 150 ml destilliertem Wasser gelöst und der pH-Wert der Lösung mit 5 N Natronlauge auf 5,8 gestellt. Danach gibt man 5,0 g rohes Thermolysin (Thermoase SP 160, Fa. DAIWA KASEI K.K.) zu. Die Lösung wird auf 40 °C thermostatisiert und unter Rühren über einen Zeitraum von 5 Stunden mit 121,5 g (0,5 Mol) D-,L-Phenylalaninpropylesterhydrochlorid versetzt, das in 100 ml destilliertem Wasser gelöst ist. Dabei wird der pH-Wert der Lösung durch Zugabe von 5N Natronlauge auf 5,8 gehalten. Nach 8 Stunden filtriert man das ausgefallene Produkt ab, wäscht mit 500 ml destilliertem Wasser nach und trocknet die Kristalle im Trockenschrank bei 70 °C.

Man erhält 126 g Addukt (95 % bezogen auf die eingesetzte N-geschützte Asparaginsäure).

Schmelzpunkt: 134 - 136 °C

Optischer Drehwert: $[\alpha]_D^{25}$: - 8,4 (1 c in Methanol)

$^1$H-NMR (100 MHz, DMSO-$d_6$):

7,4 - 7,0 (15H); 6,2 - 6,0 (5H); 5,05 (2H); 4,85 - 4,40 (2H); 4,10 - 3,85 (5H); 3,20 - 2,50 (6H); 1,85 - 1,45 (4H); 1,00 - 0,75 (6H).

Beispiel 2

Zu einer Lösung von 1 g Calciumacetat und 5,0 g Thermoase SP 160 in 100 ml destilliertem Wasser tropft man im Verlauf von 6 Stunden 53,0 g (0,2 Mol) L-N-Benzyloxycarbonylasparaginsäure und 109 g (0,45 Mol) D-,L-Phenylalaninpropylesterhydrochlorid, die in 200 ml destilliertem Wasser gelöst sind. Die Reaktionstemperatur beträgt 40 °C. Der pH-Wert der Lösung wird während der Reaktion durch Zugabe von 5N Natronlauge auf 5,8 gehalten. Nach 8 Stunden Reaktionszeit filtriert man von ausgefallenem Addukt ab und wäscht mit 300 ml Wasser nach.

Man erhält 113 g Addukt (85 % bezogen auf die eingesetzte N-geschützte Asparaginsäure).

Beispiel 3

Herstellung von N-geschütztem L-Aspartylphenylalaninbutylester-Addukt.

Man verfährt gemäß Beispiel 1 oder 2, setzt jedoch D-,L-Phenylalaninbutylesterhydrochlorid ein.
Die Ausbeuten sind analog der genannten Beispiele.

Schmelzpunkt: 124 - 128 °C

Optischer Drehwert: $[\alpha]_D^{25}$: -4,2 (1 c in Methanol)

Beispiel 4

Herstellung von N-geschütztem L-Aspartylphenylalaninpropylester

107 g (0,161 Mol) des aus Beispiel 1 oder 2 erhaltenen Addukts werden in 1 l 2N Salzsäure aufgenommen und 30 Minuten bei Raumtemperatur kräftig gerührt. Man filtriert das ausgefallene Produkt ab, wäscht gut mit destilliertem Wasser nach und trocknet im Trockenschrank bei 80 °C.

Man erhält 72 g des N-geschützten L-Aspartylphenylalaninpropylester (98,0 % d.Th.).

Schmelzpunkt: 118 °C
Optischer Drehwert: $[\alpha]_D^{25}$: - 17,O (1 c in Methanol)
$^1$H-NMR (100 MHz, DMSO-$d_6$)
8,25 - 8,15 (1H); 7,50 - 7,40 (1H); 7,30 - 7,15 (15H); 4,97 (2H); 4,60 - 4,10 (2H); 3,96 - 3,80 (2H); 3,45 - 3,25 (2H); 3,05 - 2,95 (2H); 1,65 -1,20 (4H); 0,85 - 0,70 (3H).

Beispiel 5

Herstellung von N-geschütztem L-Aspartylphenylalaninbutylester.

Man verfährt gemäß Beispiel 4, setzt jedoch das aus Beispiel 3 erhaltene Addukt ein.
Die Ausbeuten sind analog Beispiel 4.
Schmelzpunkt: 112 °C
Optischer Drehwert: $[\alpha]_D^{25}$: - 16,5 (1c in Methanol)

Beispiel 6

Rückgewinnung des Phenylalaninpropylesters

Das aus Beispiel 4 erhaltene wäßrige Filtrat wird mit 5N NaOH auf den pH-Wert 8,0 gestellt. Der Propylester wird mit Diethylether extrahiert. Nach dem Trocknen der organischen Phase wird dann durch Einleiten von gasförmiger Salzsäure der Propylester als Hydrochlorid ausgefällt.
Man erhält 33,0 g (84 % d.Th.) Phenylalaninpropylesterhydrochlorid.

Beispiel 7

Umesterung des N-geschützten L-Aspartylphenylalaninpropylester

84 g (0,18 Mol) des aus Beispiel 4 erhaltenen Produkts werden in 840 ml wasserfreiem Methanol, das über Magnesium destilliert wird, aufgelöst und unter Rühren mit 50 ml 30%iger methanolischer Natriummethanolatlösung (0,28 Mol) versetzt. Nach 17 Minuten bei Raumtemperatur ist die Reaktion vollständig abgelaufen, wie mit einem HPLC-Gerät verfolgt werden kann. Man gibt 60 ml (0,3 mol) Eisessig zu diesem Gemisch und engt die Lösung am Rotationsverdampfer ein. Der ölige Rückstand wird in Ethylacetat/n-Hexan [4/1 (vol/vol)] zur Kristallisation gebracht.
Ausbeute: 77,0 g N-geschützter L-Aspartylphenylalaninmethylester
Optischer Drehwert: $[\alpha]_D^{25}$: -14,7; (1 c in Methanol)

Beispiel 8

Man verfährt gemäß Beispiel 7, arbeitet jedoch bei 5°C. Nach 4 Stunden ist die Reaktion vollständig abgelaufen mit analogen Ergebnissen wie nach Beispiel 7.

**Patentansprüche**

1. Verfahren zur Herstellung der Verbindung der allgemeinen Formel III,

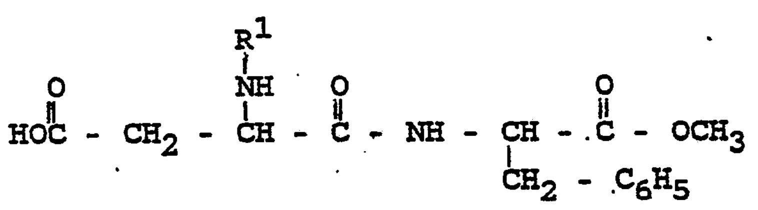 III

in der $R^1$ eine Aminoschutzgruppe ist, dadurch gekennzeichnet, daß die Verbindung der allgemeinen Formel II,

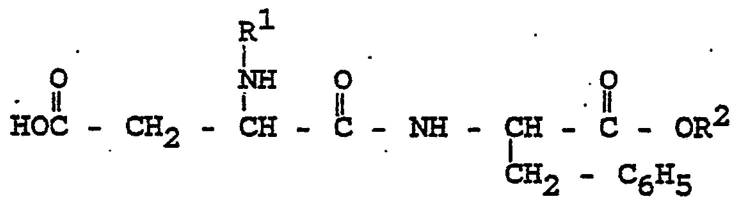 II

in der $R^1$ eine Aminoschutzgruppe und $R^2$ eine Alkylgruppe mit 3-7 Kohlenstoffatomen bedeuten, in wasserfreiem Methanol in Gegenwart eines basischen Katalysators umgeestert wird.

2. Verfahren nach Anspruch 1, dadurch gekennzeichnet, daß $R^1$ eine Aminoschutzgruppe vom Urethantyp ist.

3. Verfahren nach Anspruch 2, dadurch gekennzeicnet, daß $R^1$ eine Benzyloxycarbonylgruppe oder eine tertiär-Butyloxycarbonylgruppe ist.

4. Verfahren nach einem oder mehreren der Ansprüche 1-3, dadurch gekennzeichnet, daß $R^2$ eine n-Propyl-, iso-Propyl-, n-Butyl- oder n-Pentylgruppe ist.

5. Verfahren nach einem oder mehreren der Ansprüche 1-4, dadurch gekennzeichnet, daß die Reaktion unter Zugabe von Alkalimetallmethanolat durchgeführt wird.

6. Verfahren nach Anspruch 5, dadurch gekennzeichnet, daß die Reaktion unter Zugabe von Natriummethanolat durchgeführt wird.

7. Verfahren nach einem oder mehreren der Ansprüche 1-6, dadurch gekennzeichnet, daß das Alkalimetallalkoholat in 1,0- bis 5facher molarer Menge, bezogen auf die Verbindung der allgemeinen Formel II, eingesetzt wird.

8. Verfahren nach Anspruch 7, dadurch gekennzeichnet, daß das Alkalimetallalkoholat in der 1,1- bis 2,0fachen molaren Menge eingesetzt wird.

9. Verfahren nach einem oder mehreren der Ansprüche 1-8, dadurch gekennzeichnet, daß die Reaktion bei -20 bis 30°C durchgeführt wird.

10. Verfahren nach Anspruch 9, dadurch gekennzeichnet, daß die Reaktion bei 18 bis 25 °C durchgeführt wird.

**Claims**

1. A process for the preparation of the compound of the general formula III

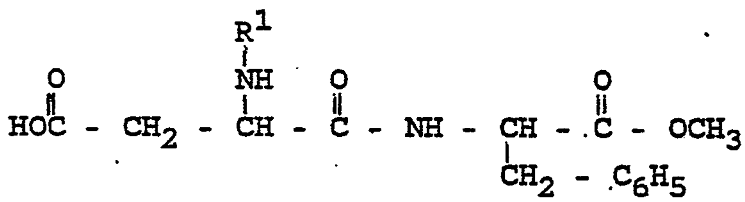 III

in which $R^1$ is an amino-protecting group, which comprises transesterifying the compound of the general formula II

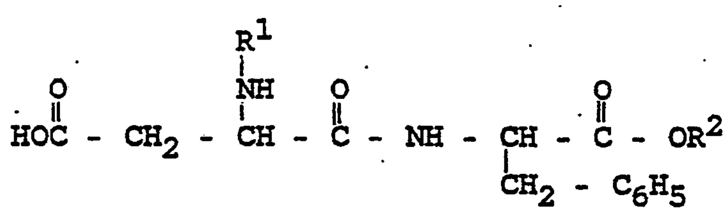 II

in which $R^1$ denotes an amino-protecting group and $R^2$ denotes an alkyl group having 3-7 carbon atoms, in anhydrous methanol in the presence of a basic catalyst.

2. The process as claimed in claim 1, wherein $R^1$ is an amino-protecting group of the urethane type.

3. The process as claimed in claim 2, wherein $R^1$ is a benzyloxycarbonyl group or a tertiary butyloxycarbonyl group.

4. The process as claimed in one or more of claims 1 - 3, wherein $R^2$ is an n-propyl, iso-propyl, n-butyl or n-pentyl group.

5. The process as claimed in one or more of claims 1 - 4, wherein the reaction is carried out with addition of alkali metal methanolate.

6. The process as claimed in claim 5, wherein the reaction is carried out with addition of sodium methanolate.

7. The process as claimed in one or more of claims 1 - 6, wherein the alkali metal alcoholate is employed in 1.0 to 5 times the molar amount, relative to the compound of the general formula II.

8. The process as claimed in claim 7, wherein the alkali metal alcoholate is employed in 1.1 to 2.0 times the molar amount.

9. The process as claimed in one or more of claims 1 - 8, wherein the reaction is carried out at -20 to 30°C.

10. The process as claimed in claim 9, wherein the reaction is carried out at 18 to 25°C.

**Revendications**

1. Procédé pour la préparation du composé de formule générale III

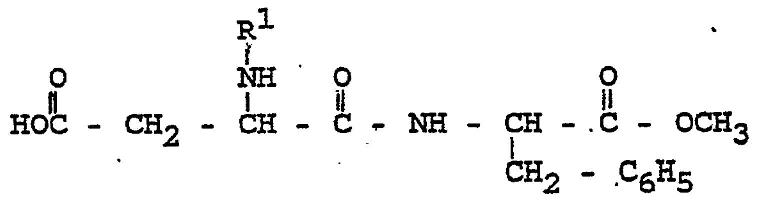 III

dans laquelle $R^1$ est un groupe protecteur de fonction amino, qui est caractérisé en ce que l'on soumet à une transestérification dans du méthanol anhydre, en présence d'un catalyseur basique, le composé de formule générale II

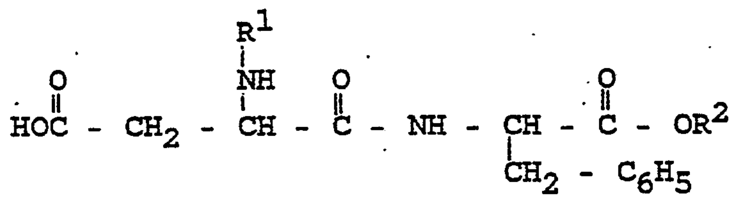 II

dans laquelle $R^1$ représente un groupe protecteur de fonction amino, et $R^2$ représente un groupe alkyle ayant de 3 à 7 atomes de carbone.

2. Procédé selon la revendication 1, caractérisé en ce que $R^1$ est un groupe protecteur de fonction amino du type uréthanne.

3. Procédé selon la revendication 2, caractérisé en ce que $R^1$ est un groupe benzyloxycarbonyle ou un groupe tert-butyloxycarbonyle.

4. Procédé selon une ou plusieurs des revendications 1 à 3, caractérisé en ce que $R^2$ est un groupe n-propyle, isopropyle, n-butyle ou n-pentyle.

5. Procédé selon une ou plusieurs des revendications 1 à 4, caractérisé en ce que la réaction est effectuée avec addition d'un méthanolate de métal alcalin.

6. Procédé selon la revendication 5, caractérisé en ce que la réaction est effectuée avec addition de méthanolate de sodium.

7. Procédé selon une ou plusieurs des revendications 1 à 6, caractérisé en ce que l'on utilise l'alcoolate de métal alcalin en une quantité de 1,0 à 5 fois molaire, par rapport au composé de formule générale II.

8. Procédé selon la revendication 7, caractérisé en ce que l'on utilise l'alcoolate de métal alcalin en une quantité de 1,1 à 2,0 fois molaire.

9. Procédé selon une ou plusieurs des revendications 1 à 8, caractérisé en ce que l'on effectue la réaction à une température de -20 à 30°C.

10. Procédé selon la revendication 9, caractérisé en ce que l'on effectue la réaction à une température de 18 à 25°C.